# EUROPEAN PATENT APPLICATION

(11) **EP 2 022 411 A1**
(43) Date of publication of application: **11.02.2009**
(21) Application number: 08252517.1
(22) Date of filing: 24.07.2008
(51) Int. Cl.: A61B 17/02

(54) **Tissue manipulator**

(30) Priority: 25.07.2007 US 782696
(71) Applicant: Ethicon Endo-Surgery, Inc., Cincinnati, OH 45242 (US)
(72) Inventor: Measamer, John P., Cincinnati, OH 45249 (US)
(74) Representative: Tunstall, Christopher Stephen

(57) **Abstract**

A needle (10) for use in a tissue manipulator is described. The needle (10) has first and second piercing arms (20,40) joined by a resilient biasing member (60), such as a spring (60). The needle (10) is formed of a super elastic material, such as a material having a stress induced martensite state, and can be deformed from a stored, unstressed configuration to a stressed, deployed configuration. A different portion of the needle (10) such as a portion of the arms (20,40) can be configured to subsequently super elastically deform if a predetermined stress level is reached in that position of the needle (10). Thus preventing tearing of the tissue.

## Description

This patent application cross references and incorporates by reference US Patent Application "Method of Tissue Manipulation" filed in the name of John Measamer on even date herewith.

### FIELD OF THE INVENTION

The present invention relates in general to medical devices, and more generally to medical devices useful for manipulating tissue.

### BACKGROUND OF THE INVENTION

Tissue manipulators can be employed to grasp and or hold tissue so that other diagnostic or treatment procedures are carried out with the tissue held in a desired position. For instance, it can be desirable to hold and manipulate tissue in the stomach while performing other procedures on the inside surface of the stomach. In many instances, it is desirable to grasp a full thickness of the stomach, including not only the inner, mucosal layer, but also the muscle layer.

One type of tissue manipulator is a grasping type tissue manipulator which uses jaws to grasp tissue. Grasping type manipulators, when used, can be ineffective in grasping a full thickness of stomach wall tissue if the inner mucosal layer separates from the muscle layer.

Another type of tissue manipulator employs a corskscrew shaped device to grasp tissue, such as is shown in US2004/0194790. Corkscrew retractors typically have a single point of insertion, which can result in high local stresses on the tissue being engaged, resulting in tearing of the tissue.

US2004/0025194, incorporated herein by reference, discloses a tissue retractor which can employ a shape memory material. The retractor disclosed has two flexible needles which can be formed of the shape memory material. The memory shape of the needles can include an arcuate shape when the needles are extended. Still, Engineers and scientists continue to seek improved ways to grasp and manipulate tissue.

### SUMMARY OF THE INVENTION

Applicant has recognized the desirability of having a tissue manipulator which can be employed in methods to reliably grasp and retract tissue, and which does not require multiple individual needles, or mechanisms to deploy multiple needles. Further, Applicant has recognized the desirability of having a tissue manipulator which has a needle stored in an unstressed, unstrained retracted configuration.

In one embodiment, the invention provides a single, unitary needle comprising a first arm having a first piercing tip, a second arm having a second piercing tip, and a resilient biasing member joining the first arm and the second arm. The resilient biasing member can be a coil spring integral with the arms, and the first and second arms can extend from opposite ends of the coil spring to have a generally narrow V shape in the stored, unstressed configuration of the needle.

The needle can be formed of a super elastic alloy material. As used herein, super elastic refers to a material that deforms reversibly to strains up to at least about nine percent, such as by creation of a stress induced material phase. For example, the needle can be formed of an alloy exhibiting a stress induced martensitic structure. In one embodiment, the needle can be formed from Nitinol, and can be deformable from the V shaped, stored configuration to a deployed, relatively flatter configuration where at least some of the material of the needle assumes a stress induced martensitic state upon deployment of the needle. The needle is reversibly deformable from the unstressed, V shaped stored configuration to the deployed, generally flatter tissue engaging configuration.

In one embodiment, the invention provides a needle for manipulating tissue, the needle formed of a super elastic alloy material, wherein the needle is reversibly deformable from an unstressed, stored configuration to a stressed, tissue engaging configuration. Preferably the needle comprises nitinol. Preferably the needle material takes on a stress induced martensitic state upon being deformed to the tissue engaging configuration. Preferably the needle comprises at least two piercing tips. Preferably the needle comprises a resilient biasing member. More preferably the resilient biasing member comprises a coil spring.

In another embodiment, a tissue manipulator is provided having an elongate body having a proximal end and a distal end; an end piece disposed at the distal end of the elongate body; and a one piece, unitary needle having at least two, generally oppositely facing piercing tips. The needle can be formed of a super elastic alloy material, and the needle can be disposed at least partially within the end piece in a generally unstressed, stored configuration. The needle can be reversibly deformable to assume a deployed configuration with the piercing tips extending from the end piece.

Preferably the needle material has a reversible stress induced martensitic state, and the needle material takes on the stress induced martensitic state upon deployment of the piercing tips from the endpiece. Preferably the needle is generally V-shaped in the stored configuration. Preferably the needle comprises a first needle arm having a first piercing tip, a second needle arm comprising a second piercing tip, and coil spring joining the first and second needle arms. Preferably the tissue manipulator further comprises an actuating member for deforming the needle, wherein the actuating member is operable to push the needle in a distal direction to deploy the first and second piercing tips from the end piece.

In another embodiment, a method is provided for engaging and retracting tissue within a patient's body, such as within a hollow organ (such as the stomach), or in a body lumen. The method can include the steps of providing a medical device comprising a needle formed of a super elastic alloy material, where the needle has first and second piercing tips and where the needle is reversibly deformable from an unstressed, stored configuration with the piercing tips disposed within a portion of the medical device to a stressed, tissue engaging configuration with the piercing tips extended from the medical device; positioning the needle in the body with the needle in the unstressed, stored configuration; deforming the needle to take on the stressed, tissue engaging configuration where the needle piercing tips are extended into tissue; and manipulating tissue with the needle while maintaining the needle in the stressed, tissue engaging configuration.

The step of positioning the needle in the body can include positioning the needle in the body with the needle material in an austenitic state, and the step of deforming the needle can include placing at least some of the needle material in a stress induced martensitic state, such as by deforming a generally sharp V-shaped needle formed of nitinol, and the step of deforming can include at least partially flattening the V-shape of the needle.

The method can also include the step of retracting the needle tips relative to a portion of the medical device, where the step of retracting comprises unloading a coil spring integrally formed with the needle.

### BRIEF DESCRIPTION OF THE DRAWINGS

While the specification concludes with claims particularly pointing out and distinctly claiming the present invention, it is believed the same will be better understood by reference to the following description, taken in conjunction with the accompanying drawings in which:

Figure 1 is a front view schematic illustration of a generally V shaped needle according to one embodiment of the present invention, with the needle shown in its unstressed, undeformed configuration.

Figure 2 is a side view schematic illustration of the needle shown in Figure 1.

Figure 3 is a front view schematic illustration of the needle of Figure 1 shown in its deformed, generally flattened configuration.

Figure 4 shows a tissue manipulator according to one embodiment of the present invention having a proximal actuation assembly, an elongate, flexible body, a distal end piece, and showing the piercing points of a needle of the type depicted in Figure 3 extending from openings in the distal end of the tissue manipulator.

Figure 5 shows a portion of the distal end piece of Figure 4 positioned against tissue within the stomach, with a portion of the distal end piece shown cut away to depict the V shaped needle stored in a retracted, unstressed configuration within the end piece, and an actuating wire engaging the coil spring at the base of the V shaped needle.

Figure 6 shows the portion of the distal end piece of Figure 5 with the actuating wire advanced distally to deform and flatten the V shaped needle, and with the piercing tips of the needle penetrating the mucosal lining of the stomach and the muscle layer of the stomach.

### DETAILED DESCRIPTION OF THE INVENTION

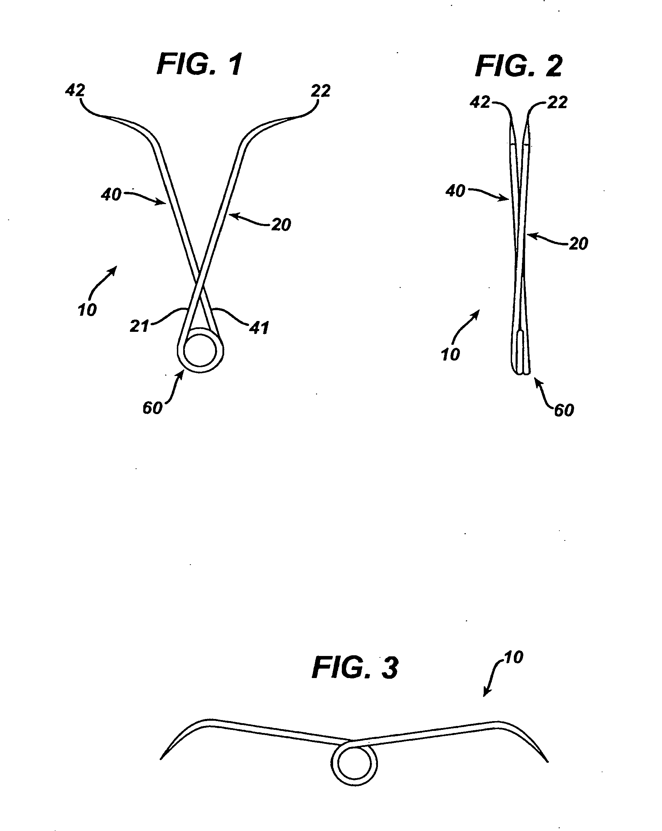

Referring to Figures 1 and 2, a needle 10 is shown having a generally V shaped configuration. The needle 10 includes a first arm 20 and a second arm 40. The first arm 20 extends from a proximal portion 21 to a distal portion which includes a first piercing tip 22, suitably sharp and pointed for penetrating tissue. Second arm 40 likewise extends from a proximal portion 41 to a distal portion which includes a second piercing tip 42. The needle 10 shown is a unitary, one piece needle. The needle 10 can be generally solid, or alternatively, can include one or more hollow portions.

Each arm 20 and 40 can include at least one generally straight portion and at least one generally curved portion. In Figure 1, the proximal portions 21 and 41 are generally straight, and the distal portions associated with piercing tips 22 and 42 are generally arcuate or hook shaped.

The needle 10 also includes a resilient biasing member, such as coil spring 60. The coil spring 60 joins the first arm 20 and the second arm 40, and can be formed integrally with arms 20 and 40, such as by forming needle 10 from a length of wire stock. The coil spring can include one or more coils, including fractional coils, depending on the desired spring restoring force and space limitations. In other embodiments, other suitable spring configurations can be used as alternatives to the coil spring 60.

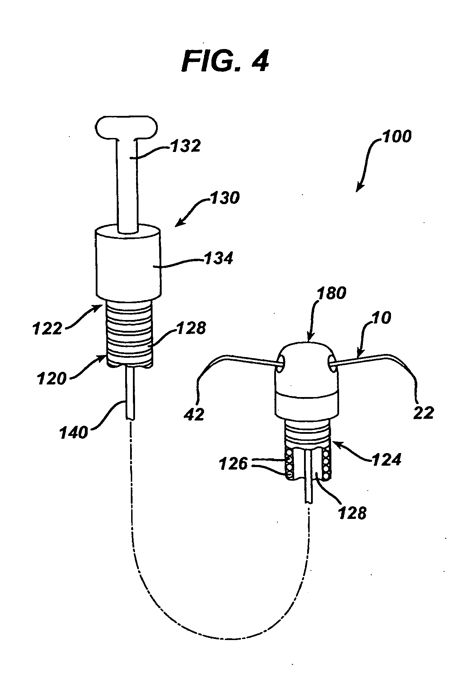

The configuration of needle 10 in Figure 1 corresponds to a relatively low energy, unstressed, stored configuration in which needle 10 can be stored in the tissue manipulator shown in Figures 4-6. Figure 3 illustrates needle 10 deformed from the stored unstressed configuration of Figure 1 to a deployed, relatively flattened configuration shown in Figure 3. In the deployed configuration of Figure 3, the spring 60 provides a biasing force which tends to restore the needle to the unstressed, stored configuration of Figure 1.

Suitable materials from which needle 10 can be formed include biocompatible metals, such as stainless steel, titanium, nickel, and alloys of nickel and titanium, as well as non-metals. For instance, needle 10 can be formed of one of the alloys described in US Patents 4,665,906 and 5,067,957 to Jervis, which patents are incorporated herein by reference. In one embodiment, the needle 10 can be formed from a super elastic material having an austenitic structure and which displays a reversible stress induced martensite structure, such as at room temperature (70 Fahrenheit) and/or body temperature (98.6 Fahrenheit).

In one embodiment, needle 10 can be formed of Nitinol, with needle the material being in the austenitic state when in the stored unstressed configuration of Figure 1. The needle can be formed such the needle material has an austenitic structure in the stored configuration, and such that at least some of the needle material is transformed to martensite upon deformation to the relatively flattened, deployed configuration of Figure 3. For instance, the needle can be designed (such as by choice of the cross sectional dimension of the winding of coil spring 60 and the heat treatment used in forming the needle 10) so that as the needle is deformed from the stored configuration to the deployed configuration, a portion of the coil spring 60 transitions to a martensitic structure and is capable of deforming reversibly, with substantially no permanent deformation, to take on the shape shown in Figure 3. If desired, the needle 10 can be configured such that some, but not all of the needle material transitions to martensite as the needle takes on the deployed configuration.

Figure 4 illustrates a tissue manipulator 100 which can be used to extend and retract needle 10. Tissue manipulator 100 can include an elongated, flexible body 120 having a proximal end 122 and a distal end 124. The elongated flexible body 120 can include a flexible coil winding 126 (shown in cut-away at the distal end of the body 120 in Figure 4). The coil winding 126 can be covered with a sheath 128. Sheath 128 can be formed of a thin film material, such as a polyolefin, polyethylene, Teflon, or other suitable sheath material.

In an alternative embodiment, the needle 10 can employed with a laparoscopic device, such as a device having a relatively rigid shaft rather than the flexible body 120, and the end piece 180 and the shaft can be sized to be insertable through a trocar.

An actuator assembly 130 is disposed at the proximal end 122 of body 120. The actuator assembly 130 can include a plunger 132 movable in an actuator body 134 joined to the proximal end 122 of the flexible body 120. The tissue manipulator can also include an end piece 180 disposed at the distal end 124 of the elongated flexible body 120.

The plunger 132 is operatively associated with a control member, such as control wire 140. The proximal end of the control wire 140 can be connected, directly or indirectly to the plunger 132. The control wire 140 extends through flexible coil winding 126 of the elongated flexible body 120, and the distal end of the control wire 140 is operatively associated with the needle 10. Plunger 132 can be advanced or withdrawn in actuator body 134 to cause needle 10 to extend from, or be retracted into, the end piece 180.

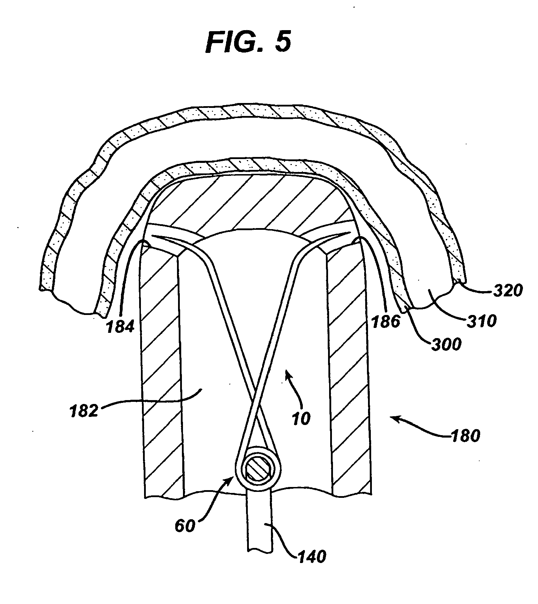

Figure 5 illustrates the distal end of the control wire 140 pivotably engaged with the coil spring 60 of the needle 10. The distal end of the control wire 140 can have a short bend (L shaped, Z shaped, or otherwise) to extend through the opening defined by coil spring 60.

Figure 5 illustrates the distal portion of the end piece 180 cut away to show the needle 10 disposed within an internal chamber 182 of end piece 180. In Figure 5, the needle 10 is in the stored, unstressed configuration. Two guide passages 184 and 186 extend from chamber 182 through the wall of the end piece 180. The guide passages can be sized and shaped to receive the arms 20 and 40 of the needle 10 as the needle is extended from end piece 180.

In use, the elongated flexible body 120 and end piece 180 can be directed into a patient's body, such as through an open surgical incision, through an endoscope, or through a laparoscope. The end piece 180 can be advanced to press against tissue, such as at a location inside the stomach, or other hollow organ or body passageway. In Figure 5, the end piece 180 is shown within the stomach, and advanced against the inside surface of the stomach. The layers of the stomach wall are indicated by reference numbers 300 (mucosa), 310 (muscle layer), and 310 (serosa).

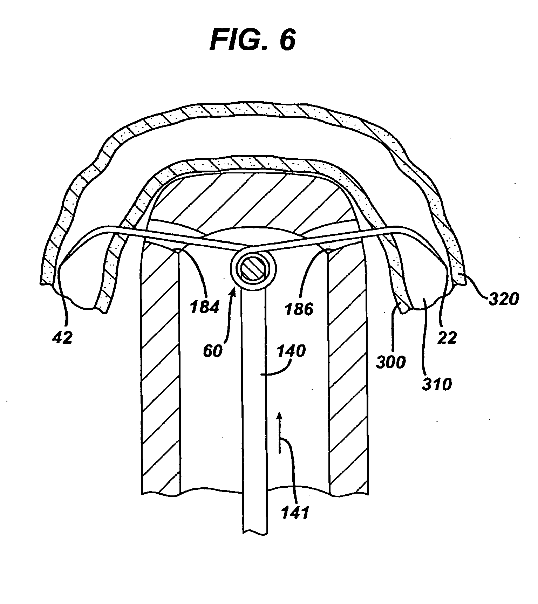

In Figure 6, the control wire 140 is shown advanced distally (as indicated by arrow 141) to advance the coil spring 60 distally, and load the spring 60 in torsion. Advancing the coils spring 60 distally causes arms 20 and 40 of needle 10 to be extended out of passageways 184 and 186 to enter the stomach wall. The distal piercing tips 22 and 42 are shown directed into the stomach muscle layer 310, providing a more reliable grip on the stomach wall than would otherwise be obtained if only the mucosal layer 300 were engaged.

To retract or otherwise manipulate tissue, the control member 140 can be held fixed relative to the end piece 180, while the end piece is advance, retracted, or rotated relative to the stomach. To release the tissue, the control member can be retracted proximally relative to end piece 180 to withdraw piercing tips 22 and 42 from the tissue.

By forming the needle from a super elastic material, such as a material having a stress induced martensite structure, the needle 10 can be tailored to limit the total pulling force that can be applied to the tissue without causing irreversible yielding of the needle. This load limiting can help prevent tearing of the tissue.

For instance, one portion of the needle (such as a portion of the coil spring 60) can be configured to super elastically deform as the needle is deployed from the tissue manipulator, and a different portion of the needle (such as a portion of the arms 20/40) can be configured to subsequently super elastically deform if a predetermined stress level is reached in that portion of the needle. In one embodiment, the needle 10 can be formed such that a portion of the needle (such as a portion of the coil spring 60) transitions to martensite as the needle is deployed, and such that another portion of the needle (such as portions of the arms 20 and 40) subsequently transitions to martensite at predetermined level of bending stress in the arms corresponding to the needle being pulled or otherwise manipulated with a force sufficient to potentially cause tissue tearing. If desired, different portions of the needle can be designed with different cross-sectional attributes (such as for instance bending moment of inertia), with different heat treatments, different alloy compositions, and combinations thereof, so that different portions of the needle 10 deform super elastically under different load applications. By way of example, the windings of the coil spring 60 can be designed with a cross sectional shape or dimension to have cross section with a bending moment of inertia that is greater than or less than the bending moment of inertia of the cross section of the arms 20,40.

For instance, one or more portions of the arms 20 and 40 can be configured (such as by choice of cross sectional shape and dimensions, and heat treatment) to transition to martensite in association with the bending stress in that portion of the arms exceeding a predetermined amount (such as a predetermined bending stress level which is associated with potential tearing of the tissue being grasped by the arms 20 and 40). Under such loading, the arms can be configured to super elastically deform (such that the arms can bend to be generally parallel to the longitudinal axis of the end piece 180) to thereby allow the tissue to slip off of the needle 10. Once the needle slips off of the tissue, the arms 20 and 40 return to their austenitic configuration and permanent shape.

The devices disclosed herein can be designed to be disposed of after a single use, or they can be designed to be used multiple times. In either case, however, the device can be reconditioned for reuse after at least one use. Reconditioning can include any combination of the steps of disassembly of the device, followed by cleaning or replacement of particular pieces, and subsequent reassembly. In particular, the device can be disassembled, and any number of the particular pieces or parts of the device can be selectively replaced or removed in any combination. Upon cleaning and/or replacement of particular parts, the device can be reassembled for subsequent use either at a reconditioning facility, or by a surgical team immediately prior to a surgical procedure. Those skilled in the art will appreciate that reconditioning of a device can utilize a variety of techniques for disassembly, cleaning/replacement, and reassembly. Use of such techniques, and the resulting reconditioned device, are all within the scope of this disclosure.

Preferably, the invention described herein will be processed before surgery. First, a new or used instrument is obtained and if necessary cleaned. The instrument can then be sterilized. In one sterilization technique, the instrument is placed in a closed and sealed container, such as a plastic or TYVEK bag. The container and instrument are then placed in a field of radiation that can penetrate the container, such as gamma radiation, x-rays, or high-energy electrons. The radiation kills bacteria on the instrument and in the container. The sterilized instrument can then be stored in the sterile container. The sealed container keeps the instrument sterile until it is opened in the medical facility.

While preferred embodiments of the present invention have been shown and described herein, it will be obvious to those skilled in the art that such embodiments are provided by way of example only. Numerous variations, changes, and substitutions will now occur to those skilled in the art without departing from the spirit and scope of the appended claims. Additionally, each element described in relation to the invention can be alternatively described as a means for performing that element's function.

## Claims

1. A needle for manipulating tissue, the needle comprising:
a first arm having a first piercing tip;
a second arm having a second piercing tip; and
a resilient biasing member joining the first arm and the second arm.

2. The needle of Claim 1 wherein the resilient biasing member comprises a spring.

3. The needle of Claim 2 wherein the resilient biasing member comprises a coil spring.

4. The needle of Claim 2 wherein the spring is integral with the first arm and the second arm.

5. The needle of Claim 1 wherein needle comprises a super elastic alloy.

6. The needle of Claim 1 wherein the needle comprises nitinol.

7. The needle of Claim 1 wherein the needle comprises a material having a stress induced martensite state.

8. The needle of Claim 7 wherein the needle is deformable from a stored configuration to a deployed configuration, and wherein at least a portion of the needle assumes a stress induced martensitic state upon being deployed.

9. The needle of Claim 1 wherein the needle is deformable from an unstressed, stored configuration to a stressed, deployed configuration.

10. A needle for manipulating tissue, the needle formed of a super elastic alloy material, wherein the needle is reversibly deformable from an unstressed, stored configuration to a stressed, tissue engaging configuration.

11. The needle of Claim 10 wherein the needle comprises nitinol.

12. The needle of Claim 10 wherein the needle material takes on a stress induced martensitic state upon being deformed to the tissue engaging configuration.

13. The needle of Claim 10 comprising at least two piercing tips.

14. The needle of Claim 10 comprising a resilient biasing member.

15. A medical device for use in manipulating tissue, the medical device comprising:
an elongate body having a proximal end and a distal end;
an end piece disposed at the distal end of the elongate body; and
a one piece needle having at least two piercing tips, wherein the needle is formed of a super elastic alloy material, wherein the needle is disposed at least partially within the end piece in a generally unstressed, stored configuration, and wherein the needle is reversibly deformable to assume a deployed configuration wherein the piercing tips extend from the end piece.
